# EUROPEAN PATENT SPECIFICATION

(11) **EP 2 425 792 B1**
(45) Date of publication and mention of the grant of the patent: **19.11.2014**
(21) Application number: 11180368.0
(22) Date of filing: 07.09.2011
(51) Int. Cl.: A61B 18/14, A61B 18/00, A61B 18/12

(54) **An electrosurgical instrument**
Elektrochirurgisches Instrument
Instrument électrochirurgical

(30) Priority: 07.09.2010 US 876731
(43) Date of publication of application: 07.03.2012
(73) Proprietor: Covidien LP, Mansfield, MA 02048 (US)
(72) Inventor: Johnson, Kristin D., Louisville, Colorado 80027 (US)
(74) Representative: Hoffmann Eitle

(56) References cited:
- EP-A1- 1 878 400
- EP-A2- 0 913 126
- WO-A1-96/05776
- US-A1- 2001 037 109
- US-A1- 2006 217 697

## Description

### BACKGROUND

### Technical Field

The present disclosure relates to an end effector assembly for an electrosurgical instrument and, more particularly, to an end effector assembly for an electrosurgical instrument configured to dissect, seal or otherwise treat tissue.

### Background of Related Art

Electrosurgical instruments, e.g., electrosurgical forceps (open type or closed type, i.e., suitable for a laparoscopic procedure), are well known in the medical arts and typically include an end effector assembly including jaw members configured to manipulate tissue (e.g., grasp and seal tissue). Typically, the electrosurgical forceps utilizes both mechanical clamping action and electrical energy to effect hemostasis by heating the tissue and blood vessels to coagulate, cauterize, seal, desiccate, and/or fulgurate tissue.

In certain instances, it may prove advantageous to cut or dissect tissue that has been electrosurgically treated, e.g., sealed. In such instances, a cutting element, e.g., a knife blade, may be configured to translate through a knife channel that is disposed on one or both of the jaw members. As can be appreciated, incorporating the knife blade into the electrosurgical instrument may increase manufacturing costs of the electrosurgical instrument. In addition, manufacturing tolerances typically associated with the placement of the knife channel on one or both of the jaw members need to be kept at a minimum. That is, the knife blade needs to be substantially aligned with the knife channel during the manufacture of the end effector and/or jaw members. As can be appreciated, if the knife blade and knife channel are not substantially aligned with each other, then during translation of the knife blade through the knife channel, the knife blade may contact the knife channel, which, in turn, may lead to tissue not being effectively severed.

In addition to electrosurgical instruments, ultrasonic instruments may be utilized to treat tissue. Conventional ultrasonic instruments, e.g., an ultrasonic dissector, typically include an end effector assembly including jaw members configured to manipulate tissue (e.g., grasp and seal tissue). Typically, the ultrasonic dissector utilizes both mechanical clamping action and ultrasonic energy to effect hemostasis by heating the tissue and blood vessels to coagulate, cauterize, seal, cut, dissect, desiccate, and/or fulgurate tissue. While ultrasonic instruments may effectively treat and, subsequently, dissect tissue, ultrasonic instruments are typically not configured to articulate and/or "flex." As can be appreciated, this limits their use in the surgical environment.

Reference is made to EP 1 878 400 A1. This document discloses a treatment device having a first jaw with a first electrode portion forming a tissue pressing portion and a second jaw with an insulating receiving member provided at a position opposite the tissue pressing portion, the receiving member positioned within a central recess of a second electrode portion of the second jaw. Both a coagulation/incision mode and a coagulation mode are disclosed.

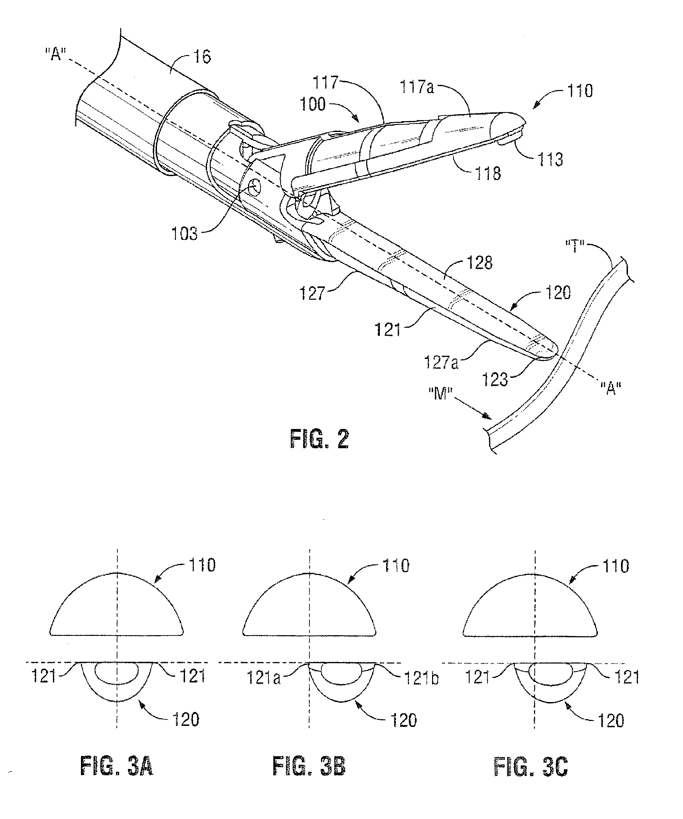

Attention is also invited to WO 96/05776, which discloses forceps that exhibit a pair of (upper and lower) jaws which contain electrodes for RF coagulation of tissue held between the jaws. In one embodiment (see Fig. 2B) the upper jaw is split lengthwise down the middle, to accommodate a cutting blade for cutting vessels following coagulation. In another embodiment (see Fig. 2C) the cutting blade is offset to one side of the upper jaw thereby to facilitate side cutting following coagulation on only one side of the area to be cut.

### SUMMARY

The invention is defined bv the independent claim below. Dependent claims are directed to optional features and preferred embodiments.

An end effector assembly for an electrosurgical instrument is disclosed. The end effector assembly has a pair of first and second jaw members including respective seal plates having a width. Each of the seal plates is adapted to connect to an energy source. One or both of the first and second jaw members may be movable relative to the other jaw member from an open position, wherein the first and the second jaw members are disposed in spaced relation relative to one another, to a clamping position, wherein the first and second jaw members cooperate to grasp tissue therebetween. The first and second jaw members are operable in two modes of operation, a first mode of operation for treating tissue and a second mode of operation for separating tissue. The seal plate of the first jaw member includes a width that is smaller than a width of the seal plate of the second jaw member. In the second mode of operation, the seal plate of the first jaw member is independently activatable from the seal plate of the second jaw member to facilitate the separation of tissue when the first and second jaw members are in one of the open and clamping positions.

In a disclosed configuration, the seal plate of the second jaw member is only partly covered by the seal plate of the first jaw member when the jaw members are in a closed position as a result of the differing widths. In an embodiment, the seal plates are of the same length. In an embodiment, the seal plates are of planar form, wherein the plane of the seal plates defines a longitudinal or length direction and a lateral or width direction. Inner and outer edges of the seal plates extend longitudinally from a proximal end to a distal end of the seal plate and define between them a lateral or width-wise extent of the seal plates. In an embodiment, the widths differ by at least 0.1 mm, 0.5 mm, 1 mm, 1.5 mm, 1.9 mm along at least a major portion of the seal plates. In an embodiment, the widths differ by 5 mm or less, 4 mm or less, 3.5 mm or less, 3 mm or less, 2.5 mm or less or 2.1 mm or less along at least a major portion of the seal plates.

A system for performing an electrosurgical procedure is disclosed. The system includes an energy source that is configured to function in two or more modes of operation, a first mode of operation for treating tissue and a second mode of operation for dissecting tissue. The system includes an electrosurgical forceps that includes a handle having a shaft that extends therefrom and defines a longitudinal axis therethrough. An end effector assembly operatively connected to a distal end of the shaft and has a pair of first and second jaw members including respective seal plates having a width. Each of the seal plates is adapted to connect to an energy source. One or both of the first and second jaw members are movable relative to the other jaw member from an open position, wherein the first and the second jaw members are disposed in spaced relation relative to one another, to a clamping position, wherein the first and second jaw members cooperate to grasp tissue therebetween. The first and second jaw members are operable the first mode of operation for treating tissue and the second mode of operation for separating tissue. The seal plate of the first jaw member includes a width that is smaller than a width of the seal plate of the second jaw member. In the second mode of operation, the seal plate of the first jaw member is independently activatable from the seal plate of the second jaw member to facilitate the separation of tissue when the first and second jaw members are in one of the open and clamping positions.

A method for performing an electrosurgical procedure is disclosed. The method includes positioning tissue between first and second jaw members of an electrosurgical instrument. The first and second jaw members are operable in two modes of operation, a first mode of operation for treating tissue and a second mode of operation for separating tissue. The seal plate of the first jaw member includes a width that is smaller than a width of the seal plate of the second jaw member. The method includes closing the first and second jaw members such that the tissue is clamped therebetween. Transmitting electrosurgical energy in the first mode of operation to the first and second jaw members for electrosurgically treating tissue is a step of the method. And, transmitting electrosurgical energy in the second mode of operation to the seal plate of the first jaw member for dissecting the electrosurgically treated tissue is another step of the method.

In a disclosed configuration, in the second mode of operation, the seal plate of the first jaw member is independently activatable from the seal plate of the second jaw member to facilitate the separation of tissue when the first and second jaw members are in one of the open and clamping positions.

### BRIEF DESCRIPTION OF THE DRAWINGS

Embodiments are described hereinbelow with reference to the drawings wherein:
FIG. 1 is a left, perspective view of an electrosurgical instrument including an end effector having jaw members (not in accordance with the present invention);
FIG. 2 is an enlarged, left perspective view of the indicated area of detail of FIG. 1 with a dissecting jaw member adjacent tissue (not in accordance with the present invention);
FIGS. 3A-3C are front views of the jaw members depicted in FIG. 2 illustrating various alignment configurations thereof (FIG. 3A is not in accordance with the present invention);
FIG. 4 is side view of jaw members depicted in FIG. 2 with tissue positioned across the dissecting jaw member; and
FIG. 5 is side view of jaw members depicted in FIG. 2 with tissue positioned across the dissecting jaw member with the jaw members in a clamping position.

### DETAILED DESCRIPTION OF THE EMBODIMENTS

Detailed embodiments are disclosed herein; however, the disclosed embodiments are merely examples, which may be embodied in various forms. Therefore, specific structural and functional details disclosed herein are not to be interpreted as limiting, but merely as a basis for the claims and as a representative basis for teaching one skilled in the art to variously employ the present disclosure in virtually any appropriately detailed structure.

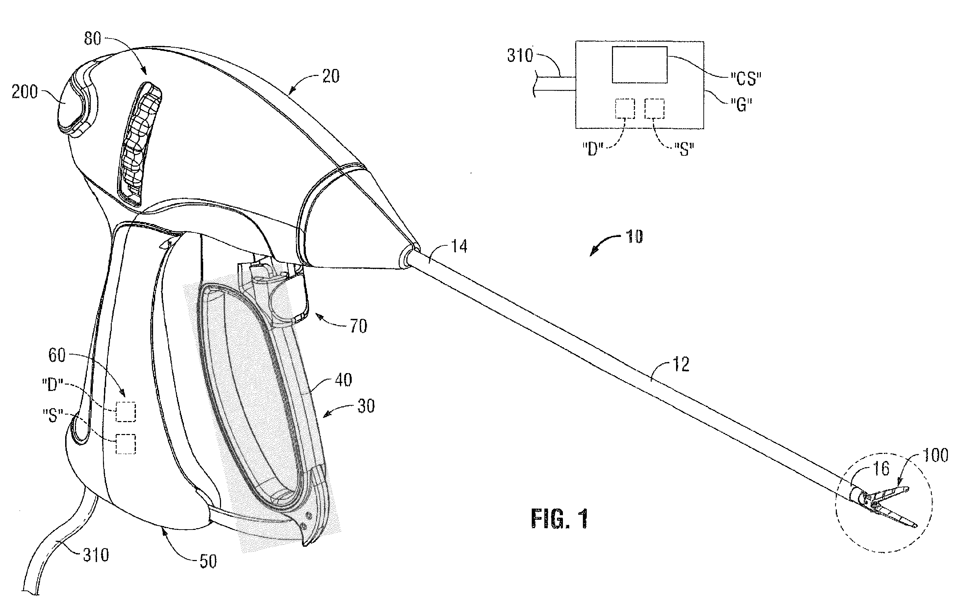

Turning now to FIGS. 1-4, and initially with reference to FIG. 1, an electrosurgical instrument, e.g., an electrosurgical forceps 10 (forceps 10), that includes an end effector 100, not in accordance with the present invention. is shown. Forceps 10 is operatively and selectively coupled to an electrosurgical generator (generator "G") for performing an electrosurgical procedure (FIG. 1). In some instances, the forceps 10 may be battery-powered. For purposes herein, an electrosurgical procedure may include sealing, cutting, dissecting, cauterizing, coagulating, desiccating, and/or fulgurating tissue all of which may employ RF energy. The generator "G" is configured for monopolar and bipolar modes of operation. The generator "G" may include or is in operative communication with a control system "CS" (FIG. 1) that may include one or more processors in operative communication with one or more control modules that are executable on the processor. The control module (not explicitly shown) may be configured to instruct one or more modules to transmit electrosurgical energy, which may be in the form of a wave or signal/pulse, via one or more cables (e.g., a cable 310) to one or both jaw members 110 and 120 of an end effector 100.

Briefly, forceps 10 is configured for use with various surgical procedures and includes a housing 20. A shaft 12 extends distally from the housing 20 and defines a longitudinal axis "A-A" therethrough. In the drawings and in the descriptions that follow, the term "proximal," as is traditional, will refer to the end of the forceps 10 that is closer to the user, while the term "distal" will refer to the end that is farther from the user. The shaft has a distal end 16 configured to mechanically engage the end effector assembly 100 and a proximal end 14 that mechanically engages the housing 20. In certain instances, the shaft 12 may be configured to bend or articulate. For example, shaft 12 may be resilient or portion thereof may include an articulating member.

A handle assembly 30 includes a fixed handle 50 and a movable handle 40. Fixed handle 50 is integrally associated with housing 20 and handle 40 is movable relative to fixed handle 50. In certain configurations, movable handle 40 of handle assembly 30 may be operably coupled to a drive assembly (not shown), which together may be configured to cooperate to impart movement of one or both of jaw members 110 and 120 to move from an open position, wherein the jaw members 110 and 120 are disposed in spaced relation relative to one another, to a clamping or closed position, wherein the jaw members 110 and 120 cooperate to grasp tissue therebetween. Although the figure drawings depict a forceps 10 for use in connection with endoscopic surgical procedures, the present disclosure may be used for more traditional open surgical procedures. The open version of the forceps may also include the same or similar operating components and features as described below.

For a more detailed description of the housing 20, shaft 12, handle assembly 30 (including movable and fixed handles 40 and 50, respectively), rotating assembly 80, trigger assembly 70 and electrosurgical cable 310 (including line-feed configurations and/or connections), reference is made to commonly-owned U.S. Patent No. 7,150,097 to Sremcich filed June 13, 2003.

With continued reference to FIG. 1, one or more buttons or switches 60 are operably disposed on the forceps 10. More particularly, and in the illustrated configuration, two switches "D" and "S" are shown operably disposed on the fixed handle 50. In certain configurations, it may prove advantageous to provide the switches 60 on the generator "G". This of course will depend on the contemplated uses of a manufacturer. Switches "D" and "S" are in operative communication with the generator "G" and/or control system "CS" and are configured to place the forceps 10 in one or more modes of operation. More particularly, switch "S" is configured to place the forceps 10 in a first mode of operation for treating tissue, e.g., sealing tissue, and switch "D" is configured to place the forceps 10 in a second mode of operation for separating tissue, e.g., dissecting tissue.

In the first mode of operation the generator "G" including control system "CS" and the forceps 10 are configured to fuse, seal, coagulate and/or fulgurate tissue. To this end, the jaw members 110 and 120 are configured to function in a bipolar mode of operation. That is, respective seal plates 118 and 128 of jaw members 110 and 120 are both active, include opposing polarities and are configured to transmit electrosurgical energy, e.g., current, therebetween. In the second mode of operation the generator "G" including control system "CS" and the forceps 10 are configured to dissect, cut, severe and/or transect tissue. To this end, the jaw members 110 and 120 are configured to function in a monopolar mode of operation. That is, seal plate 128 is active, seal plate 118 is inactive or neutral (and/or is highly resistive to current flow), and seal plate 128 is configured to transmit electrosurgical energy, e.g., current, to tissue. In the monopolar mode of operation, a return pad or electrode is positioned on a patient and is configured to provide a return path for the current back to the generator "G".

With reference to FIG. 2, an end effector assembly 100 including jaw members 110 and 120, not in accordance with the present invention, is illustrated. In the illustrated configuration, jaw members 110 and 120 are of the unilateral type. That is, jaw member 110 is movable, e.g., pivotable, with respect to jaw member 120. Alternatively, jaw members 110 and 120 may be of the bilateral type. That is, each of the jaw members 110 and 120 are movable with respect to each other. In one arrangement, to facilitate pivoting the jaw member 110 with respect to jaw member 120, a pivot pin 103 couples the jaw members 110 and 120 to the distal end 16 of the shaft 12, as best seen in FIG. 2. Jaw members 110 and 120, and operative components associated therewith, may be formed from any suitable material, including but not limited to metal, metal alloys, plastic, plastic composites, and so forth.

Continuing with reference to FIG. 2, jaw member 110 is shown including a jaw housing 117 having a width of suitable proportion, i.e., a width that is suitable to support the seal plate 118. Electrically conductive seal plate 118 is operably supported on and secured to jaw housing 117. More particularly, a distal end 117a of jaw member 110 may be configured to securely engage the electrically conductive seal plate 118 or, with respect to a monolithic jaw member, form the seal plate 1 18.

One or more insulative or non-conductive standoffs 113 (one insulative standoff is shown) made of any suitable material, e.g., plastic, ceramic, etc., is operably disposed on the seal plate 118. More particularly, the insulative standoff 113 is operably disposed on a seal surface of the seal plate 118 at a distal end thereof, as best seen in FIG. 2. Insulative standoff 113 may be secured to the seal surface of the seal plate 118 by one or more suitable securement methods, e.g., an adhesive. In the illustrated embodiment, a "pocket" is etched in the seal surface during the manufacture process thereof, a bead of adhesive is placed in the "pocket" and the insulative standoff 113 is positioned therein. Other securement methods are contemplated.

The insulative standoff 113 is configured to contact a distal tip of the seal plate 128 when the jaw members 110 and 120 are in the clamping position such that a gap distance of suitable proportion is present between the seal surface of the seal plate 118 and a seal surface of a seal plate 128 of the jaw member 120. As a result thereof, the jaw members 118 and 128 only contact at their respective tips. Having the insulative standoff 113 positioned at the distal end of the seal plate minimizes any negative effects that may be associated with a non-conductive member being positioned on the seal surface of the seal plate 128. That is, having a portion of the seal surface of the seal plate 118 that does not conduct electrosurgical energy may compromise a tissue seal, e.g., the tissue seal may not be uniform and/or consistent across a length thereof. A uniform and/or consistent tissue seal is important, especially in the instance where one jaw member, e.g., jaw member 120, includes a seal plate 128 having a width that is smaller or "finer" than the other seal plate, e.g., seal plate 118. That is, the width of the tissue seal achieved with the jaw members 110 and 120 of the present disclosure is smaller (and thus the overall area of the tissue seal is smaller) than widths of tissue seals typically achieved by conventional jaw members.

Unlike conventional electrosurgical forceps that include end effectors having jaw members with seal plates having the same width, seal plates 118 and 128 of respective jaw members 110 and 120 of end effector 100 have different widths. More particularly, to facilitate separating tissue during the second mode of operation, the seal plate 128 of the jaw member 120 includes a width that is small in comparison to the width of the seal plate 118 of the jaw member 110. That is, seal plate 128 of the jaw member 120 is smaller or "finer" than the seal plate 118 of the jaw member 110 (see FIG. 2 in combination with FIGS. 3A-3C). For illustrative purposes, a width of the jaw housing 127 of the jaw member 120 is also illustrated as being smaller than a width of the jaw housing 117 of the jaw member 110. In some configurations, it may prove advantageous to have the jaw housing 117 and 127 with the same widths and the seal plates 118 and 128 with different widths. The specific configuration, e.g., widths, of the jaw housing 117 and 127 may be varied for a specific surgical procedure, specific manufacturer requirement, etc. Seal plate 118 8 of the jaw member 110 (the larger jaw) may includes a width that is approximately 1 mm to 2 mm larger than the width of the seal plate 128 of the jaw member 120 (the smaller or "finer" jaw member). Keeping the width of the seal plate 128 1 mm to 2 mm smaller than the width of the seal plate 117 improves visualization and dissection capabilities for the end user, e.g., a surgeon. In the illustrated configuration, seal plate 128 of the jaw member 120 includes a width that ranges from about 1 mm to about 3.4 mm and seal plate 118 of the jaw member 110 includes a width that ranges from about 3.5mm to about 5mm.

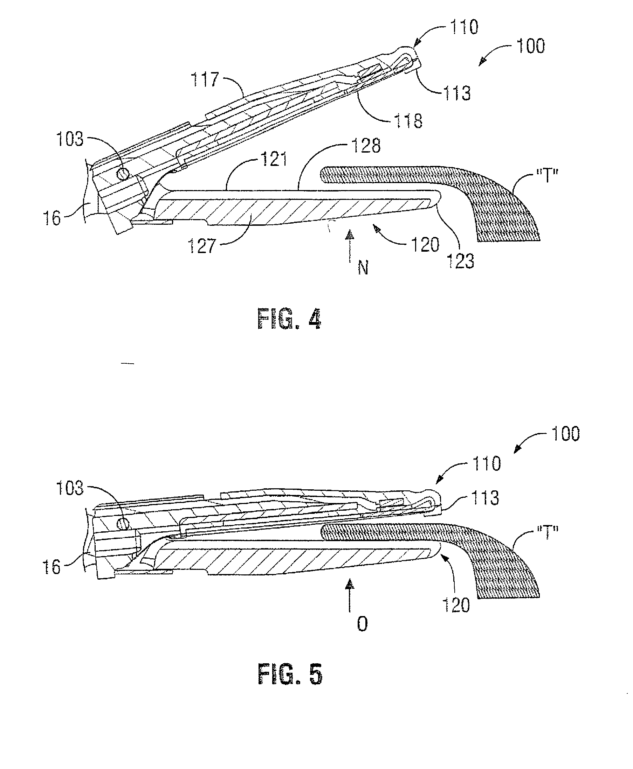

Similar to jaw member 110, jaw member 120 includes a jaw housing 127 having a distal end 127a that is configured to support seal plate 128 (FIG. 2). Unlike jaw member 110, jaw member 120 includes a seal plate 128 that includes a peripheral edge 121 that extends along a side surface of the jaw housing 127 to a distal tip 123 thereof, see FIGS. 2, 4 and 5. The peripheral edge 121 including the distal tip 123 is configured to separate tissue, e.g., dissect tissue, when the jaw members 110 and 120 are in either the open position (FIGS. 2 and 4) or the closed position (FIG. 5) and when tissue is positioned adjacent thereto. More particularly, and in one particular configuration, when switch "D" is activated, the forceps 10 is configured to operate in the second mode of operation, e.g., a monopolar mode of operation. In the second mode of operation, the generator "G" transmits electrosurgical energy to the seal plate 128 including the peripheral edge 121 and the distal tip 123 such that a user may dissect tissue that has been electrosurgically treated.

Referring to FIGS. 3A-3C, to facilitate treating and/or separating tissue, jaw members 110 and 120 may be aligned along their center lines, i.e., centrally aligned along a common axis, e.g., longitudinal axis "A-A" (FIGS. 2 and 3A, not in accordance with the present invention); aligned along a right or left portion of the peripheral edge 121 (FIG. 3B); or aligned somewhere therebetween (FIG. 3C). In the illustrated configuration, the jaw members 110 and 120 are centrally aligned along the longitudinal axis "A-A," as best seen in FIGS. 2 and 3A. Alignment along the longitudinal axis "A-A" facilitates dissecting tissue from either side of the forceps 10. In the embodiment where jaw members 110 and 120 are aligned along the right or left portion the peripheral edge 121 (see FIG. 3B where the jaw members 110 and 120 are aligned the left portion of the peripheral edge), the peripheral edge 121 includes an inner edge 121a that is configured to decrease current densities thereabout for either fusing, sealing, coagulating or fulgurating tissue during the first mode and an outer edge 121b that is configured to increase current densities thereabout for either dissecting, cutting, severing or transecting tissue during the second mode. With these purposes in mind, inner edge 121a includes a radius that is larger than a radius of the outer edge 121b, as best seen in FIG. 3B.

As can be appreciated, in any of the foregoing alignment configurations of the jaw members 110 and 120, the peripheral edge 121 (and/or edges 121a and 121b) may include radii dimensioned to accommodate a specific surgical procedure, specific manufacturer preference, etc.

Operation of forceps 10 is described in terms of use of a method for electrosurgically treating tissue, such as, for example, during a hysterectomy, a colectomy and/or a Nissen fundoplication, commonly referred to in the art as a lap Nissen. Initially, the forceps 10 is inserted through an incision in a patient. Tissue is positioned between the jaw members 110 and 120. In the instance where a user wants to seal tissue, the user activates switch "S." Activation of switch "S" indicates to the generator "G" and/or control system "CS" that the jaw members 110 and 120 are ready to operate in the bipolar mode of operation. Thereafter, generator "G" delivers electrosurgical energy to the respective seal plates 118 and 128 of the jaw members 110 and 120 to seal tissue positioned between the jaw members 110 and 120.

To dissect tissue, a user activates switch "D." Activation of switch "D" indicates to the generator "G" and/or control system "CS" that the jaw members 110 and 120 are ready to operate in the monopolar mode of operation. A return pad or electrode may be positioned (at some time prior to operation of the forceps 10 in the monopolar mode) on the patient and functions as described above. Alternatively, and in some configurations, the seal plate 118 may function as the return pad. In the monopolar mode of operation, generator "G" delivers electrosurgical energy to the seal plate 128 including the peripheral edge 121 and the distal tip 123 to dissect the electrosurgically treated tissue. During dissection, the jaw members 110 and 120 may be in either the open or closed position. Moreover, any portion of the seal plate 128 and/or the peripheral edge 121 including the distal tip 123 may be utilized to dissect the electrosurgically treated tissue.

For example, and in one particular surgical scenario, the jaw members 110 and 120 may be in the open position and the distal tip 123 may utilized to dissect the electrosurgically treated tissue. In this instance, the distal tip 123 is positioned adjacent tissue and moved in a direction indicated by directional arrow "M" into the tissue with a force of suitable proportion while simultaneously energizing the seal plate 128 (FIG. 2).

In another surgical scenario, the jaw members 110 and 120 may be in the open position and seal plate 128 may be utilized to dissect the electrosurgically treated tissue. In this instance, the seal plate 128 is positioned adjacent tissue and moved in a direction indicated by directional arrow "N" across the tissue with a force of suitable proportion while simultaneously energizing the seal plate 128 (FIG. 4).

In yet another surgical scenario, the jaw members 110 and 120 may be, initially, in the open position and seal plate 128 may utilized to dissect the electrosurgically treated tissue. In this instance, the seal plate 128 is positioned adjacent tissue and moved in a direction indicated by directional arrow "O" across the tissue with a force of suitable proportion while simultaneously energizing the seal plate 128 and closing the jaw members 110 and 120 (FIG. 5).

The forceps 10 including the jaw members 110 and 120 overcome some of aforementioned shortcomings of the above-referenced electrosurgical and/or ultrasonic instruments. More particularly, providing the forceps 10 with the finer seal plate 128 having the peripheral edge 121 eliminates the need for a knife blade and components associated therewith to dissect tissue. As can be appreciated, this lowers manufacturing costs of the forceps 10 and/or decreases or eliminates the manufacturing tolerances that are typically associated with conventional forceps. Moreover, while not discussed in great detail, the shaft 12 may be configured to bend or articulate; this provides a surgeon with greater flexibility with respect to treating and/or dissecting tissue when compared to ultrasonic instruments.

From the foregoing and with reference to the various figure drawings, those skilled in the art will appreciate that certain modifications can also be made to the present disclosure without departing from the scope of the same. For example, in certain configurations it may prove useful to have one or both of the seal plates 118 and 128 with a textured or otherwise treated seal surface.

It is contemplated that rather than configuring one of the seal plates 118 and 128 to dissect tissue, a separate or additional device may be utilized to dissect tissue. For example, one or both of the jaw members 110 and 120 may include a second or auxiliary conductive surface. More particularly, a conductive surface (not shown) of suitable proportion may be operably disposed on one or both of an exterior surface of the jaw housing 117 and 127. For example, a conductive surface may extend along a length of a bottom exterior surface of the jaw housing 127 or a conductive surface may extend along a length of a top exterior surface of the jaw housing 117. As can be appreciated, in either of these instances, the conductive surface is configured to function substantially similar to that of the seal plate 128 described above.

It is contemplated that the generator "G" may be configured to automatically detect when to place the forceps 10 in either the first or second modes of operation. In this instance, switches 60 may be utilized in a limited capacity or eliminated altogether.

## Claims

1. An end effector assembly (100) for an electrosurgical instrument (10), comprising:
a pair of first (120) and second (110) jaw members including respective seal plates having a width, the seal plates adapted to connect to an energy source (G), at least one of the first and second jaw members movable relative to the other jaw member from an open position, wherein the first and the second jaw members are disposed in spaced relation relative to one another, to a clamping position, wherein the first and second jaw members cooperate to grasp tissue therebetween, the first and second jaw members operable in two modes of operation, a first mode of operation for treating tissue and a second mode of operation for separating tissue,
wherein the width of the seal plate (128) of the first jaw member (120) is smaller than the width of the seal plate (118) of the second jaw member (110) such that in the second mode of operation the seal plate of the first jaw member is independently activatable from the seal plate of the second jaw member to facilitate the separation of tissue when the first and second jaw members are in one of the open and clamping positions,
wherein the first and second jaw members are either aligned along a peripheral edge (121) of the seal plate of the first jaw member or somewhere between being aligned along the peripheral edge and being centrally aligned along a common axis,
wherein the peripheral edge (121) includes an inner edge (121a) and an outer edge (121b), the inner edge (121a) including a radius that is larger than a radius of the outer edge (121b).

2. An end effector assembly according to claim 1, wherein the first mode of operation includes one of fusing, sealing, coagulating and fulgurating tissue and the second mode of operation includes one of dissecting, cutting, severing and transecting tissue.

3. An end effector assembly according to claim 1 or 2, wherein the seal plate of the first jaw member includes a width that ranges from about 1mm to about 3.4 mm and the seal plate of the second jaw member includes a width that ranges from about 3.5mm to about 5mm.

4. An end effector assembly according to claim 1, 2 or 3, wherein the seal plate of the first jaw member includes a peripheral edge (121) extending along a side surface of the first jaw member to a distal tip (123) thereof, the peripheral edge including the distal tip configured to separate tissue upon actuation when the first and second jaw members are in one of the open and clamping position.

5. An end effector assembly according to any one of the preceding claims, wherein a peripheral edge of the seal plate of the first jaw member includes an inner edge (121) configured to decrease current densities thereabout for one of fusing, sealing, coagulating and fulgurating tissue during the first mode of operation and an outer edge (121b) configured to increase current densities thereabout for one of dissecting, cutting, severing and transecting tissue during the second mode of operation.

6. An end effector assembly according to any one of the preceding claims, wherein a peripheral edge of the seal plate includes an inner edge including a radius that is larger than a radius of an outer edge.

7. An end effector assembly according to any one of the preceding claims, wherein the second jaw member includes at least one insulative standoff (113) configured to maintain a specific gap distance between the first and second jaw members when the first and second jaw members are in the clamping position.

8. An end effector assembly according to any one of the preceding claims, wherein the at least one insulative standoff is operably disposed at a distal end of the second jaw member and is configured to contact a distal tip of the first jaw member when the first and second jaw members are in the clamping position.

9. A system for performing an electrosurgical procedure, comprising:
an energy source (G) configured to function in at least two modes of operation, a first mode of operation for treating tissue and a second mode of operation for dissecting tissue;
an electrosurgical instrument comprising:
a handle having at least one shaft that extends therefrom that defines a longitudinal axis therethrough; and
an end effector assembly according to any one of the preceding claims and operatively connected to a distal end of the at least one shaft

10. A system for performing an electrosurgical procedure according to claim 9, wherein the energy source is a single electrosurgical generator that is configured to operate in bipolar and monopolar modes of operation.

11. A system for performing an electrosurgical procedure according to claim 9 or 10, wherein an actuation device is operably associated with one of the electrosurgical generator and the electrosurgical instrument and is configured to switch the electrosurgical instrument between the first and second modes of operation.

12. A system for performing an electrosurgical procedure according to claim 9, 10 or 11, wherein an actuation device (60) is operably disposed on the handle of the electrosurgical instrument and includes a first switch for manually placing the electrosurgical instrument into the first mode of operation and a second switch for manually placing the electrosurgical instrument into the second mode of operation.

## Patentansprüche

1. Endeffektoraufbau (100) für ein elektrochirurgisches Instrument (10), mit:
einem Paar aus einem ersten (120) und einem zweiten (110) Backenelement, die jeweils eine Versiegelungsplatte mit einer Breite aufweisen, wobei die Versiegelungsplatten angepasst sind, mit einer Energiequelle (G) verbunden zu sein, mindestens eines des ersten und zweiten Backenelements relativ zu dem anderen Backenelement von einer offenen Position, in der das erste und zweite Backenelement in einer beabstandeten Beziehung relativ zueinander angeordnet sind, zu einer Klammerposition bewegbar ist, in der das erste und zweite Backenelement zusammenwirken, um zwischen sich Gewebe zu greifen, das erste und zweite Backenelement in zwei Betriebsmodi betriebsfähig sind, einem ersten Betriebsmodus zum Behandeln von Gewebe und einem zweiten Betriebsmodus zum Trennen von Gewebe,
wobei die Breite der Versiegelungsplatte (128) des ersten Backenelements (120) schmaler ist als die Breite der Versiegelungsplatte (118) des zweiten Backenelements (110), sodass in dem zweiten Betriebsmodus die Versiegelungsplatte des ersten Backenelements unabhängig von der Versiegelungsplatte des zweiten Backenelements aktivierbar ist, um die Trennung von Gewebe zu ermöglichen, wenn das erste und zweite Backenelement in der offenen oder der Klammerposition sind,
wobei das erste und zweite Backenelement entweder entlang einer Umfangskante (121) der Versiegelungsplatte des ersten Backenelements oder irgendwo zwischen dem entlang der Umfangskante ausgerichtet sein und dem mittigen entlang einer gemeinsamen Achse angeordnet sein,
wobei die Umfangskante (121) eine innere Kante (121a) und eine äußere Kante (121b) aufweist, wobei die innere Kante (121a) einen Radius aufweist, der größer ist als ein Radius der äußeren Kante (121b).

2. Endeffektoraufbau nach Anspruch 1, bei dem der erste Betriebsmodus Verschweißen, Versiegeln, Koagulieren oder Elektroblitzbehandeln von Gewebe aufweist und der zweite Betriebsmodus Auseinanderschneiden, Schneiden, Abtrennen oder Durchtrennen von Gewebe aufweist.

3. Endeffektoraufbau nach Anspruch 1 oder 2, bei dem die Versiegelungsplatte des ersten Backenelements eine Breite aufweist, die sich in etwa 1 mm bis in etwa 3,4 mm bewegt und die Versiegelungsplatte des zweiten Backenelements eine Breite aufweist, die sich von in etwa 3,5 mm bis in etwa 5 mm bewegt.

4. Endeffektoraufbau nach Anspruch 1, 2 oder 3, bei dem die Versiegelungsplatte des ersten Backenelements eine Umfangskante (121) aufweist, die sich entlang einer Seitenfläche des ersten Backenelements zu einer distalen Spitze (123) davon erstreckt und die Umfangskante die distale Spitze aufweist, die eingerichtet ist, bei Betätigung Gewebe zu trennen, wenn das erste und zweite Backenelement in der offenen Position oder Klammerposition sind.

5. Endeffektoraufbau nach einem der vorstehenden Ansprüche, bei dem eine Umfangskante der Versiegelungsplatte des ersten Backenelements eine innere Kante (121) aufweist, die eingerichtet ist, für das Verschweißen, Versiegeln, Koagulieren oder Elektroblitzbehandeln von Gewebe während des ersten Betriebsmodus Stromdichten dort herum zu vermindern, und eine äußere Kante (121b) aufweist, die eingerichtet ist, für das Auseinanderschneiden, Schneiden, Abtrennen oder Durchtrennen von Gewebe während des zweiten Betriebsmodus Stromdichten dort herum zu erhöhen.

6. Endeffektoraufbau nach einem der vorstehenden Ansprüche, bei dem eine Umfangskante der Versiegelungsplatte eine innere Kante einschließlich eines Radius aufweist, der größer ist als ein Radius einer äußeren Kante

7. Endeffektoraufbau nach einem der vorstehenden Ansprüche, bei dem das zweite Backenelement mindestens ein isolierendendes Abstandselement (113) aufweist, das eingerichtet ist, einen bestimmten Spaltabstand zwischen dem ersten und zweiten Backenelement aufrechtzuerhalten, wenn das erste und zweite Backenelement in der Klammerposition sind.

8. Endeffektoraufbau nach einem der vorstehenden Ansprüche, bei dem das mindestens eine isolierende Abstandselement betriebsfähig bei einem distalen Ende des zweiten Backenelements angeordnet ist und eingerichtet ist, eine distale Spitze des ersten Backenelements zu berühren, wenn das erste und zweite Backenelement in der Klammerposition sind.

9. System zum Ausführen eines elektrochirurgischen Eingriffs, mit:
einer Energiequelle (G), die eingerichtet ist, in zumindest zwei Betriebsmodi zu arbeiten, einem ersten Betriebsmodus zum Behandeln von Gewebe und einem zweiten Betriebsmodus zum Auseinanderschneiden von Gewebe;
einem elektrochirurgischen Instrument, das aufweist:
einen Griff mit einem Schaft, der sich von diesem erstreckt, der dort hindurch eine Längsachse definiert; und
einen Endeffektoraufbau nach einem der vorstehenden Ansprüche und betriebsfähig mit einem distalen Ende des mindestens einen Schafts verbunden.

10. System zum Ausführen eines elektrochirurgischen Eingriffs nach Anspruch 9, bei dem die Energiequelle ein einziger elektrochirurgischer Generator ist, der eingerichtet ist, in einem bipolaren und einem monopolaren Betriebsmodus zu arbeiten.

11. System zum Ausführen eines elektrochirurgischen Eingriffs nach Anspruch 9 oder 10, bei dem eine Betätigungseinrichtung betriebsfähig mit dem elektrochirurgischen Generator oder dem elektrochirurgischen Instrument zusammenhängt und eingerichtet ist, das elektrochirurgische Instrument zwischen dem ersten und dem zweiten Betriebsmodus umzuschalten.

12. System zum Ausführen eines elektrochirurgischen Eingriffs nach Anspruch 9, 10 oder 11, bei dem eine Betätigungseinrichtung (60) betriebsfähig an dem Griff des elektrochirurgischen Instruments angeordnet ist und einen ersten Schalter zum manuellen Platzieren des elektrochirurgischen Instruments in dem ersten Betriebsmodus und einen zweiten Schalter zum manuellen Platzieren des elektrochirurgischen Instruments in dem zweiten Betriebsmodus aufweist.

## Revendications

1. Ensemble d'effecteur terminal (100) pour un instrument électro-chirurgical (10), comprenant :
une paire de premier (120) et de deuxième (110) éléments de mâchoire incluant des plaques d'obturation respectives ayant une largueur, les plaques d'obturation étant aptes à être connectées à une source d'énergie (G), au moins un des premier et deuxième éléments de mâchoire étant déplaçables relativement à l'autre élément de mâchoire d'une position ouverte, où les premier et deuxième éléments de mâchoire sont disposés en une relation espacée l'un relativement à l'autre, à une position de serrage, où les premier et deuxième éléments de mâchoire coopèrent pour saisir le tissu entre eux, les premier et deuxième éléments de mâchoire étant aptes à fonctionner selon deux mode d'opération, un premier mode d'opération pour le traitement du tissu et un deuxième mode d'opération pour la séparation du tissu,
où la largueur de la plaque d'obturation (128) du premier élément de mâchoire (120) est plus petite que la largueur de la plaque d'obturation (118) du deuxième élément de mâchoire (110) de sorte que dans le deuxième mode d'opération, la plaque d'obturation du premier élément de mâchoire peut être activée indépendamment de la plaque d'obturation du deuxième élément de mâchoire pour faciliter la séparation du tissu lorsque les premier et deuxième éléments de mâchoire se trouvent dans l'une des positions ouverte et de serrage,
où les premier et deuxième éléments de mâchoire sont soit alignés le long d'un bord périphérique (121) de la plaque d'obturation du premier élément de mâchoire ou sont alignés quelque part le long du bord périphérique et étant alignés centralement le long d'un axe commun,
où le bord périphérique (121) comprend un bord intérieur (121a) et un bord extérieur (121b), le bord intérieur (121a) incluant un rayon qui est plus grand qu'un rayon du bord extérieur (121b).

2. Ensemble effecteur terminal selon la revendication 1, dans lequel le premier mode de fonctionnement comprend un parmi la fusion, l'obturation, la coagulation et la fulguration du tissu, et le deuxième mode de fonctionnement comprend un parmi la dissection, la coupe, la séparation et la transsection du tissu.

3. Ensemble effecteur terminal selon la revendication 1 ou 2, dans lequel la plaque d'obturation du premier élément de mâchoire présente une largueur qui s'étend d'environ 1mm à environ 3,4mm, et la plaque d'obturation du deuxième élément de mâchoire présente une largueur qui s'étend d'environ 3,5mm à environ 5mm.

4. Ensemble effecteur terminal selon la revendication 1, 2 ou 3, dans lequel la plaque d'obturation du premier élément de mâchoire comprend un bord périphérique (121) s'étendant le long d'une surface latérale du premier élément de mâchoire à une pointe distale (123) de celui-ci, le bord périphérique incluant la pointe distale configurée pour séparer le tissu lors de l'actionnement lorsque les premier et deuxième éléments de mâchoire se trouvent dans une parmi les positions ouverte et de serrage.

5. Ensemble effecteur terminal selon l'une quelconque des revendications précédentes, dans lequel un bord périphérique de la plaque d'obturation du premier élément de mâchoire présente un bord intérieur (121) configuré pour réduire les densités du courant autour de celui-ci pendant l'une parmi la fusion, l'obturation, la coagulation et la fulguration du tissu durant le premier mode de fonctionnement, et un bord extérieur (121b) configuré pour augmenter les densités du courant autour de celui-ci pour l'une parmi la dissection, la coupe, la séparation et la transsection du tissu durant le deuxième mode de fonctionnement.

6. Ensemble effecteur terminal selon l'une quelconque des revendications précédentes, dans lequel un bord périphérique de la plaque d'obturation comprend un bord intérieur incluant un rayon qui est plus grand qu'un rayon d'un bord extérieur.

7. Ensemble effecteur terminal selon l'une quelconque des revendications précédentes, dans lequel le deuxième élément de mâchoire comprend au moins une pièce d'écartement isolante (113) configurée pour maintenir une distance d'espace spécifique entre les premier et deuxième éléments de mâchoire lorsque les premier et deuxième éléments de mâchoire sont dans la position de serrage.

8. Ensemble effecteur terminal selon l'une quelconque des revendications précédentes, dans lequel au moins une pièce d'écartement isolante précitée est fonctionnellement disposée à une extrémité distale du deuxième élément de mâchoire et est configurée pour venir en contact avec une pointe distale du premier élément de mâchoire lorsque les premier et deuxième éléments de mâchoire sont dans la position de serrage.

9. Système pour exécuter une procédure électro-chirurgicale, comprenant :
une source d'énergie (G) configurée pour fonctionner selon au moins deux modes de fonctionnement, un premier mode de fonctionnement pour le traitement du tissu et un deuxième mode de fonctionnement pour la dissection du tissu ;
un instrument électro-chirurgical comprenant :
une poignée ayant au moins un arbre qui s'étend de celle-ci qui définit un axe longitudinal à travers celle-ci ; et
un ensemble effecteur terminal selon l'une quelconque des revendications précédentes et fonctionnellement connecté à une extrémité distale d'au moins un arbre précité.

10. Système pour exécuter une procédure électro-chirurgicale selon la revendication 9, dans lequel la source d'énergie est un générateur électro-chirurgical unique qui est configuré pour fonctionner en mode bipolaire et mono-polaire d'opération.

11. Système pour exécuter une procédure électro-chirurgicale selon la revendication 9 ou 10, dans lequel un dispositif d'actionnement est fonctionnellement associé à l'un parmi le générateur électro-chirurgical et l'instrument électro-chirurgical et est configuré pour commuter l'instrument électro-chirurgical entre les premier et deuxième modes de fonctionnement.

12. Système pour exécuter une procédure électro-chirurgicale selon la revendication 9, 10 ou 11, dans lequel le dispositif d'actionnement (60) est fonctionnellement disposé sur la poignée de l'instrument électro-chirurgical et comprend un premier commutateur pour placer manuellement l'instrument électro-chirurgical en premier mode de fonctionnement, et un deuxième commutateur pour placer manuellement l'instrument électro-chirurgical en deuxième mode de fonctionnement.
